# EUROPEAN PATENT APPLICATION

(11) **EP 1 619 240 A1**
(43) Date of publication of application: **25.01.2006**
(21) Application number: 04725820.7
(22) Date of filing: 05.04.2004
(51) Int. Cl.: C12M 3/00, C12N 5/00

(54) **INCUBATOR AND METHOD OF CELL CULTURING**

(30) Priority: 09.04.2003 JP 2003104877
(71) Applicant: Yamashita, Kikuji, Myozai-gun, Tokushima 779-3215 (JP); Ishikawa, Tomoyasu, Tokushima 7700861 (JP)
(72) Inventor: Yamashita, Kikuji, Myozai-gun, Tokushima 779-3215 (JP); Ishikawa, Tomoyasu, Tokushima 7700861 (JP)
(74) Representative: Wagner, Karl H.
(86) International application number: PCT/JP2004/004930
(87) International publication number: WO 2004/090092

(57) **Abstract**

It is an object to provide an incubator capable of reliably maintaining a culture condition to be constant and reducing the weight and size of an apparatus, and effectively preventing a cell from being contaminated by bacteria or the like, and a cell culturing method capable of promoting the growth of a cell and the differentiation of an undifferentiated cell.

In an apparatus including environment regulating means having a culture space 1h which can be sealed and serving to maintain an internal environment in the culture space 1h into a predetermined state, the environment regulating means of the apparatus includes temperature regulating means 10 for regulating a temperature in the culture space 1h, the temperature regulating means 10 having a far infrared ray radiating portion for radiating a far infrared ray.

## Description

### Technical Field

The present invention relates to an incubator and a cell culturing method. The cell of an organism or the like is generally cultured in a sealed container, that is, a so-called CO₂ incubator on a condition that a temperature, a humidity and the partial pressures of oxygen and carbon dioxide are maintained to be constant in order to prevent a contamination or an infection from being caused by bacteria, mold or the like.

The present invention relates to an incubator capable of reducing a change in an internal environment and culturing a cell on a stable condition.

In the culture of the cell, moreover, a growth factor is caused to act on the cell to be cultured, thereby promoting the growth of the cell or a differentiation-inducing factor is caused to act on an undifferentiated cell, thereby promoting the differentiation of the cell.

The present invention relates to a method of culturing a cell utilizing a far infrared ray as a factor for promoting the differentiation or growth of the cell.

### Background Art

An incubator which has conventionally been used comprises a culture chamber capable of being sealed from an outside thereof, and the internal wall of the culture chamber is provided with a water jacket for functioning as a heat insulator. Therefore, it is possible to prevent the temperature of a gas in the culture chamber from fluctuating by the influence of outside air. Therefore, the temperature of a cell which is raised by a heat transfer from the gas in the culture chamber can be maintained to be constant.

In a process for culturing a cell, however, it is necessary to grasp the state of the cell periodically. For this purpose, the cell which is being cultured is to be periodically taken out of the culture chamber, and to be inspected and analyzed. In order to take out the cell, the door of the incubator or the like is to be opened. At that time, the outside air flows into the culture chamber. Consequently, the temperature of the gas in the culture chamber is changed. In the case in which various bacteria are present in the outside air which flows in, there is a possibility that the cell might be contaminated by the bacteria which flow in.

As a technique for solving the problems, there have been disclosed incubators according to conventional examples 1 and 2 (Patent Documents 1 and 2).

The incubator according to the conventional example 1 is provided with internal delivery means for delivering a sample through a delivery outlet/inlet from a sample housing portion in which the cell is disposed during the culture. The internal delivery means can deliver only the sample. Therefore, it is preferable to set the delivery outlet/inlet to have such a size that the container accommodating the sample can pass therethrough, and the size of the delivery outlet/inlet can be reduced. When the sample is put in/out, consequently, the outside air flowing into the incubator can be lessened. Therefore, it is possible to suppress a change in the temperature of the gas in the culture chamber and to reduce a probability that the bacterial or the like might enter.

In the incubator according to the conventional example 2 (Patent Document 2), moreover, a circulating passage for circulating a gas in the culture chamber is provided therein and an ultraviolet lamp is disposed as a bactericidal lamp in the circulating passage. For this reason, if the gas in the culture chamber is circulated through the circulating passage, the ultraviolet rays emitted from the ultraviolet lamp can be irradiated on the gas. Therefore, it is possible to kill the bacteria contained in the gas.

### [Patent Document 1]

Japanese Laid-Open Patent Publication No. 11-89559

### [Patent Document 2]

Japanese Laid-Open Patent Publication No. 2000-166536

However, the incubator according to the conventional example 1 includes the internal delivery means. Therefore, it is possible to lessen the inflow of the outside air which is caused when the sample is put in/out. On the other hand, the incubator itself becomes large-sized and complicated. Therefore, there is a problem in that a weight is increased and the incubator is hard to handle.

In the incubator according to the conventional example 2, if the gas in the culture chamber is circulated through the circulating passage, the bacteria can be killed. However, a time taken for killing all bacteria flowing in together with the outside air is required to some degree. If the bacteria stick to the cell while the gas is circulated, they cannot be killed. For this reason, the cell is contaminated. If a light is directly irradiated on the cell or the like from the ultraviolet lamp, however, there is a problem in that the cell on which the ultraviolet ray is irradiated is also killed together with the bacteria.

Furthermore, it can be supposed that both of the incubators according to the conventional examples 1 and 2 maintain heat insulating properties with the outside by a water jacket. In order to maintain the insulating properties to be high, however, the amount of water to be accommodated in the water jacket is to be increased. Consequently, the size of the water jacket is increased. Therefore, the size of the incubator itself is to be also increased. If the amount of the water to be accommodated in the water jacket is increased, it is a matter of course that the weight of the incubator is also increased. Accordingly, it is necessary to form a large installation space for disposing the incubator. In order to provide the incubator, it is necessary to form a special table capable of supporting the weight or the like. Consequently, there is a problem in that an installation cost is also increased.

A large amount of water is accommodated in the water jacket. Therefore, a long time is required for activating the incubator. In the case in which the temperature of water is reduced for some reason during the culture, there is a problem in that a very long time is taken for raising the inside of the incubator to have a predetermined temperature before a return.

### Disclosure of the Invention

### (Object of the Invention)

In consideration of the circumstances, it is an object of the present invention to provide an incubator capable of reliably maintaining a culture condition to be constant, reducing the weight and size of an apparatus and effectively preventing a cell from being contaminated by bacteria or the like, and a cell culturing method capable of promoting the growth of the cell and the differentiation of an undifferentiated cell.

### (Structure of the Invention)

A first aspect of the present invention is directed to an incubator comprising environment regulating means having a culture space which can be sealed and serving to maintain an internal environment in the culture space into a predetermined state, the environment regulating means including temperature regulating means for regulating a temperature in the culture space, the temperature regulating means having a far infrared ray radiating portion for radiating a far infrared ray.

In the first aspect of the present invention, a second aspect of the present invention is directed to the incubator, comprising environment regulating means having a culture space which can be sealed and serving to maintain an internal environment in the culture space into a predetermined state, the environment regulating means of the apparatus including sterilizing means for carrying out a sterilization in the culture space, the sterilizing means having a far infrared ray radiating portion for radiating a far infrared ray.

In the first or second aspect of the present invention, a third aspect of the present invention is directed to the incubator, wherein the far infrared ray radiating portion is regulated to have a peak of an energy density of a far infrared ray to be radiated in a waveband of 7 to 12µm.

In the first, second or third aspect of the present invention, a fourth aspect of the present invention is directed to the incubator, wherein the far infrared ray radiating portion is a planar heating member.

In the fourth aspect of the present invention, a fifth aspect of the present invention is directed to the incubator, wherein the planar heating member is constituted by a pair of upper and lower base materials formed by a material having a waterproofness, a radiating portion sealed in a fluidtightness between the upper and lower base materials and serving to radiate a far infrared ray when it is conducted, and power supply means for supplying a power to the radiating portion, and the radiating portion is formed on the internal surfaces of the upper and lower base materials by printing a radiating member.

In the fifth aspect of the present invention, a sixth aspect of the present invention is directed to the incubator, wherein the radiating member is a material having a positive temperature coefficient.

In the fifth or sixth aspect of the present invention, a seventh aspect of the present invention is directed to the incubator, wherein the power supply means is constituted by a covering portion formed integrally with the upper and lower base materials and having a tip extended to an outside of the culture space, and a conducting portion provided from the tips of the pair of covering portions to the radiating portion, and the conducting portion is formed on the internal surfaces of the upper and lower covering portions by printing a material having a conductivity, and a portion between a tip portion and the radiating portion is sealed in a fluidtightness between the pair of covering portions.

In the first, second, third, fourth, fifth, sixth or seventh aspect of the present invention, an eighth aspect of the present invention is directed to the incubator, wherein the culture space of the apparatus is provided with a sample holding member on which a culturing object is to be disposed, and the sample holding member includes the far infrared ray radiating portion.

In the eighth aspect of the present invention, a ninth aspect of the present invention is directed to the incubator, wherein the sample holding member is removably attached to the apparatus.

A tenth aspect of the present invention is directed to a cell culturing method for culturing a cell to be cultured in a state in which a temperature thereof is held in a predetermined temperature region, wherein a far infrared ray is irradiated on the cell.

In the tenth aspect of the present invention, an eleventh aspect of the present invention is directed to the cell culturing method, wherein the cell is an animal cell, a viral infection cell or a gene recombination cell.

In the tenth aspect of the present invention, a twelfth aspect of the present invention is directed to the cell culturing method, wherein the cell is an undifferentiated cell or a juvenile cell and has a pluripotency or a differentiation potency.

In the tenth aspect of the present invention, a thirteenth aspect of the present invention is directed to the cell culturing method, wherein the undifferentiated cell is a stem cell derived from a bone marrow, a peripheral blood, a cord blood or a tissue, a cell in a differentiating process or having a differentiation potency, or an embryonic stem cell.

A fourteenth aspect of the present invention is directed to a blood product comprising material and immaterial components of a blood which is cultured, including a whole blood preparation, an erythrocyte preparation, a blood plasma preparation, a blood platelet preparation, a blood coagulation factor preparation No. VIII, an albumin preparation, an immunoglobulin preparation and the like, and the material and immaterial components of the blood are obtained by differentiating and producing the hematopoietic stem cell by an irradiation of a far infrared ray on the hematopoietic stem cell.

### (Effect of the Invention)

According to the first aspect of the present invention, if the cell to be cultured or the like is provided in the culture space, it is possible to seal the cell to be cultured or the like from the outside. In addition, an internal environment in the culture space is maintained into a predetermined state by the environment regulating means. Therefore, it is possible to reliably culture the cell to be cultured or the like in a suitable state for a growth and a differentiation. Moreover, the temperature of the cell is raised directly or through steam or the like by a radiation through a far infrared ray. Consequently, it is possible to hold the cell to have a temperature which is almost equal to that of the far infrared ray radiating portion without the influence of the temperature of a gas around the cell. Consequently, it is not necessary to extremely increase heat insulating properties between the culture space and the outside. Therefore, a water jacket does not need to be provided. Thus, it is possible to reduce the size and weight of the apparatus. It is not necessary to worry about a change in the temperature of the gas due to the inflow of the outside air. For this reason, it is not necessary to provide a special device in the culture space.

According to the second aspect of the present invention, if the cell to be cultured or the like is disposed in the culture space, it is possible to seal the cell to be cultured or the like from the outside. In addition, the internal environment in the culture space is maintained into a predetermined state by the environment regulating means. Therefore, it is possible to reliably culture the cell to be cultured or the like in a suitable state for the growth and the differentiation. Moreover, it is possible to kill the bacteria contained in the gas in the culture chamber through a far infrared ray irradiated from the far infrared ray radiating portion. Therefore, the cell which is being cultured can be prevented from being contaminated by the bacteria or the like. In addition, the far infrared ray is used. Therefore, a bad influence is not given even if the far infrared ray is directly irradiated on the cell which is being cultured. Therefore, the bacteria sticking to the cell can also be caused to die. Thus, the cell which is being cultured can be prevented more reliably from being contaminated.

According to the third aspect of the present invention, it is possible to promote the growth and differentiation of the cell through the far infrared ray. Therefore, it is possible to enhance the culturing efficiency of the cell.

According to the fourth aspect of the present invention, the far infrared ray radiating portion is the planar heating member. Therefore, it is possible to constitute the apparatus to be much more compact.

According to the fifth aspect of the present invention, if a power is supplied to the radiating portion by the power supply means, the far infrared ray can be radiated from the radiating portion. In addition, the radiating member is printed on the internal surfaces of a pair of upper and lower base members, thereby forming the radiating portion. Therefore, the planar heating member can easily be manufactured inexpensively. Moreover, the radiating portion is sealed in a fluidtightness between the upper and lower base members. Even if the radiating portion is used for a long time on a condition that a humidity in the incubator is very high, it is possible to prevent the generation of a leakage and a short circuit and to irradiate a far infrared ray on a cell in a stable state.

According to the sixth aspect of the present invention, the radiating member is a material having a positive temperature coefficient, that is, has a self-temperature regulating function. Therefore, the state of a far infrared ray to be radiated on the cell can be maintained to be constant. Accordingly, it is possible to prevent a change in the state of the far infrared ray to be irradiated on the cell by a variation in the temperature of the far infrared ray radiating portion. Consequently, it is possible to prevent a change in the temperature of the cell and to culture the cell in a stable state.

According to the seventh aspect of the present invention, the covering portion of the power supply means is formed integrally with the upper and lower base members. Therefore, it is possible to reliably seal the connecting portion of the power supply means and the radiating portion in a fluidtightness from an outside. Even if the humidity in the culture space approximates to 100%, consequently, it is possible to reliably prevent water from soaking into the radiating portion. Moreover, the conducting portion can be formed together with the radiating portion. Therefore, it is possible to decrease the manufacturing man-hour of the planar heating member and to carry out manufacture inexpensively.

According to the eighth aspect of the present invention, it is possible to directly supply a far infrared ray to the cell without air from the far infrared ray radiating portion provided on the sample holding member. Therefore, the temperature of the cell can be maintained to be constant more reliably. In addition, it is possible to decrease the amount of the far infrared ray to be absorbed in the air. Therefore, it is possible to increase an energy efficiency. Moreover, the far infrared ray can be directly supplied from the far infrared ray radiating portion to the cell. Therefore, it is possible to regulate the wavelength of the far infrared ray to be supplied from the far infrared ray radiating portion every cell. Also in a culture space, consequently, it is possible to culture the cell on different culture conditions.

According to the ninth aspect of the present invention, it is possible to regulate the area of the culture space by adjusting the number of the sample holding members.

According to the tenth aspect of the present invention, the temperature of the cell is raised by the far infrared ray. Therefore, it is possible to hold the temperature of the cell to be almost equal to that of the far infrared ray irradiating member without the influence of the temperature of the gas around the cell. Accordingly, it is possible to reliably control the temperature of the cell by simply controlling the far infrared ray radiating member. In addition, it is possible to kill bacteria contained in the gas around the cell and bacteria sticking to the cell by the killing effect of the far infrared ray. Consequently, it is possible to reliably prevent the contamination of the cell which is being cultured.

According to the eleventh aspect of the present invention, it is possible to culture almost all of the cells and to promote the growth and differentiation of the cell having a large number of differentiation potencies.

According to the twelfth aspect of the present invention, the differentiation of undifferentiated cells is promoted by the far infrared ray. Therefore, desirable differentiated cells can easily be cultured in a large amount from the differentiated cells.

According to the thirteenth aspect of the present invention, the differentiation of the stem cell is promoted by the far infrared ray. Therefore, it is possible to easily culture a desirable differentiated cell such as a blood cell in a large amount.

In the blood product according to the fourteenth aspect of the present invention, the differentiation of a hematopoietic stem cell is promoted by the far infrared ray. Therefore, it is possible to easily culture a desirable differentiated cell such as a blood cell in a large amount. Accordingly, it is possible to inexpensively manufacture a product having a desirable material component in a large amount.

### Brief Description of the Drawings

Fig. 1 is a schematic perspective view showing an incubator 1 according to the present embodiment.
Fig. 2 is a schematic front view showing the incubator 1 according to the present embodiment.
Fig. 3 is a schematic cross-sectional view showing the incubator 1 according to the present embodiment.
Fig. 4 is a schematic view showing the simple body of a planar heating member 11 to be used in the incubator 1 according to the present embodiment, (A) being a schematic plan view and (B) being a sectional view taken along a line B - B in (A).
Fig. 5 is a schematic explanatory view showing a sample holding member 5 to be used in the incubator 1 according to the present embodiment, (A) being a plan view showing a simple body, (B) being an enlarged sectional view showing a connecting portion 5a, and (C) being an enlarged view showing a connecting portion in a state in which the sample holding member 5 is connected to a body 2.
Fig. 6 is a chart showing a comparison in the increase and decrease of the number of red blood cells in the case in which the incubator according to the present invention and a general incubator are used to culture a hematopoietic stem cell.
Fig. 7 is a chart showing a comparison in the increase and decrease of the number of cells of a hemoglobin in the case in which the incubator according to the present invention and the general incubator are used to culture the hematopoietic stem cell.
Fig. 8 is a chart showing a comparison in the increase and decrease of the number of white blood cells in the case in which the incubator according to the present invention and the general incubator are used to culture the hematopoietic stem cell.
Fig. 9 is a chart showing a comparison in the increase and decrease of the number of cells of a blood platelet in the case in which the incubator according to the present invention and the general incubator are used to culture the hematopoietic stem cell.
Fig. 10 is a chart showing a comparison in the increase and decrease of the number of adhesive cells in the case in which the incubator according to the present invention and the general incubator are used to culture the adhesive cell together with the hematopoietic stem cell.

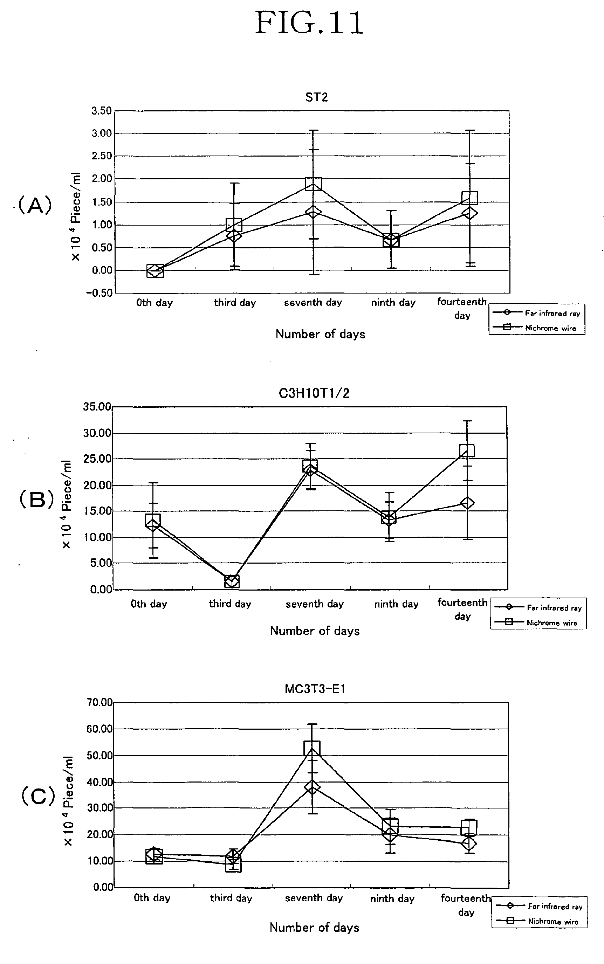

Fig. 11 is a chart showing a comparison in the increase and decrease of the number of cells in the case in which the incubator according to the present invention and the general incubator are used to culture three kinds of cells including ST-2, MC3T3-E1 and C3H10T1/2 to be mouse osteoblasts.

### Best Mode for Carrying Out the Invention

Next, an embodiment of the present invention will be described with reference to the drawings.

An incubator according to the present invention is an apparatus to be used for culturing a cell such as a stem cell derived from a bone marrow, a peripheral blood, a cord blood or a tissue, a cell in a differentiating process or having a differentiation potency, or an embryonic stem cell in a state in which a predetermined culture condition is maintained, and is characterized in that environment regulating means for maintaining the culture condition includes a far infrared ray radiating portion for radiating a far infrared ray.

First of all, description will be given to the summary of the incubator according to the present invention.

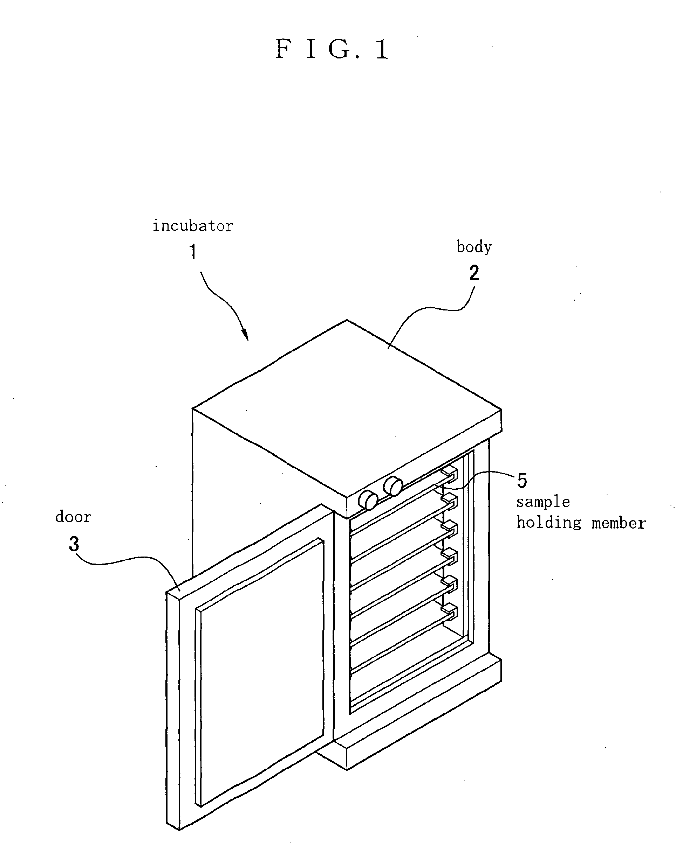

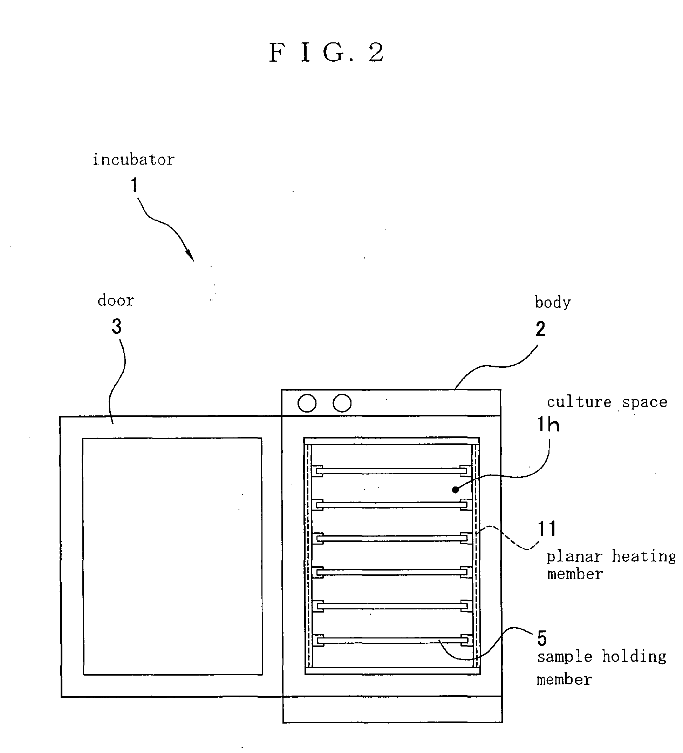

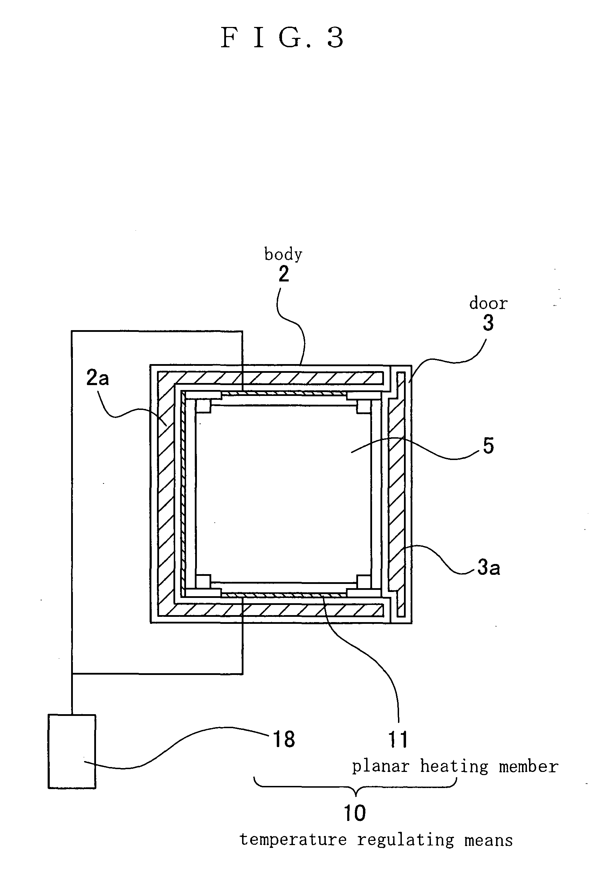

Fig. 1 is a schematic perspective view showing an incubator 1 according to the present embodiment. Fig. 2 is a schematic front view showing the incubator 1 according to the present embodiment. Fig. 3 is a schematic cross-sectional view showing the incubator 1 according to the present embodiment. As shown in Figs. 1 to 3, the incubator 1 according to the present embodiment comprises a body 2 having a culture space 1h therein and a door 3 attached openably to the body 2, and has such a structure that the culture space 1h of the body 2 is sealed in an airtightness from the outside when the door 3 is closed.

The body 2 is provided with gas exchanging means which is not shown. The gas exchanging means includes a gas supply portion such as a gas cylinder or a compressor which supplies, into the culture space 1h of the body 2, a culturing gas in which the concentrations of air and carbon dioxide or the concentrations of oxygen, nitrogen and carbon dioxide are maintained to have predetermined gas partial pressures, and a gas discharging portion such as a compressor which discharges the gas in the culture space 1h and maintains an air pressure in the culture space 1 to be constant.

Moreover, the body 2 is provided with temperature regulating means 10 for regulating the temperature of the air in the culture space 1h and humidifying means such as a water vat or a spray which regulates the humidity of the air. As shown in Fig. 3, the body 2 is provided with a heat insulator 2a such as urethane or sponge in order to surround the culture space 1h. Moreover, the door 3 is also provided with a heat insulator 3a such as a plastic inner door in order to block a portion between the culture space 1h of the body 2 and an outside when the door 3 is closed.

With the structure described above, the incubator 1 according to the present embodiment can maintain an internal environment in the culture space 1h of the body 2 into a desirable state if operating the gas exchanging means, the temperature regulating means 10 and the humidifying means with the door 3 closed. Therefore, it is possible to culture a cell disposed in the culture space 1h of the body 2 or the like in a suitable environment for a predetermined culture in an isolating state from the outside.

The gas exchanging means, the temperature regulating means 10, the humidifying means and the heat insulators 2a and 3a constitute environment regulating means described in claims.

The temperature regulating means 10 according to the present embodiment will be described in detail.

As shown in Figs. 2 and 3, a planar heating member 11 of the temperature regulating means 10 is provided on the internal wall of the culture space 1h of the body 2. The planar heating member 11 is connected to a control portion 18 for regulating a power to be supplied to the planar heating member 11. The control portion 18 serves to regulate the power to be supplied to the planar heating member 11 and to adjust the temperature of the planar heating member 11 corresponding to the culture space 1h or the temperature of the cell to be cultured.

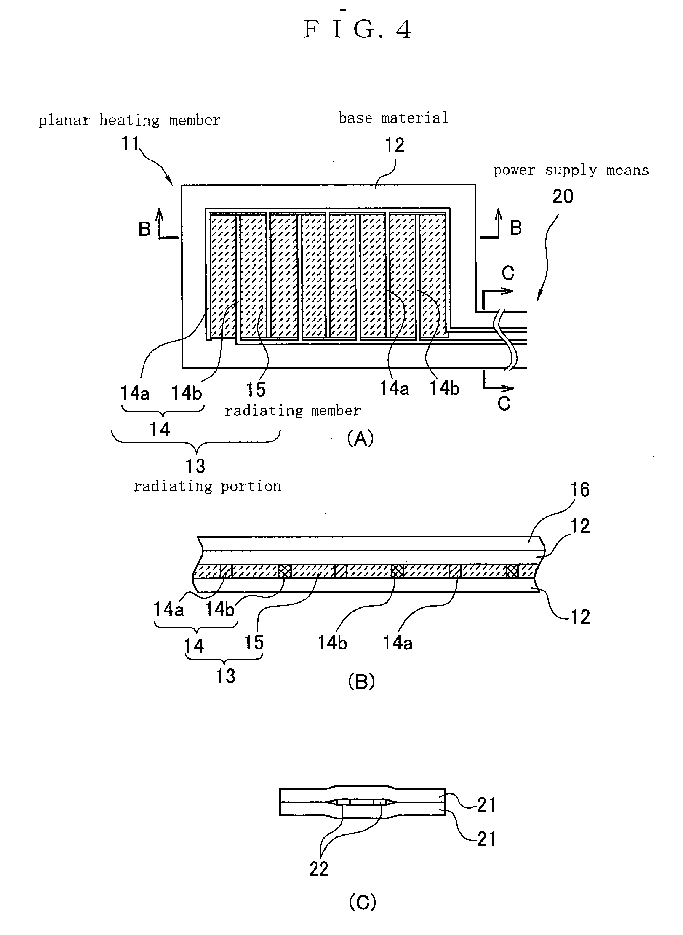

The planar heating member 11 includes a radiating portion 13 for generating heat when it is turned ON and discharging a far infrared ray toward the inner part of the culture space 1h (see Fig. 4) . In other words, the planar heating member 11 can heat a gas in the culture space 1h by heat generated by the radiating portion 13 as well as a far infrared ray discharged in the generation of the heat.

In addition, the far infrared ray radiated from the planar heating member 11 is directly propagated from the planar heating member 11 to the cell to be cultured by means of a radiating mechanism without using the gas in the culture space 1h for a medium. In this case, the energy of the far infrared ray is directly supplied to the cell.

Therefore, the temperature of the cell to be cultured can be raised by the far infrared ray in addition to a heat transfer from the gas. If the far infrared ray is irradiated on the culture, consequently, it is possible to suppress a change in the temperature of the cell itself even if the temperature of the gas around the cell to be cultured is changed. Thus, it is not necessary to greatly worry about a change in the temperature of the gas in the culture space 1h due to the inflow of outside air by opening or closing the door 3. Consequently, it is not necessary to provide a special device for delivering, into the culture space 1h, a cell which is being cultured. Therefore, it is possible to simplify the structure of the incubator 1.

Moreover, the change in the temperature of the gas around the cell rarely influences the change in the temperature of the cell itself. For this reason, it is not necessary to extremely increase the heat insulating properties between the culture space 1h and the outside. Consequently, it is not necessary to provide the water jacket for a heat insulation differently from the conventional incubator. Even if the general heat insulators 2a and 3a are used, it is possible to sufficiently suppress the change in the temperature of the cell itself. Consequently, it is possible to reduce the size and weight of the apparatus.

If the temperature of the gas in the culture space 1h is once raised to a predetermined temperature, furthermore, there is decreased an energy to be absorbed into the gas which is discharged in the configuration of a far infrared ray from the planar heating member 11. Consequently, the energy discharged as the far infrared ray from the planar heating member 11 is supplied to the cell of which most part is cultured and is used for raising the temperature of the cell itself. The energy of the far infrared ray depends on the temperature of the planar heating member 11 . Therefore, the temperature of the cell which is to be raised by the far infrared ray can be raised to be almost equal to the temperature of the planar heating member 11.

Moreover, the peak of the energy density of the far infrared ray radiated from the surface of the planar heating member 11 is regulated to be formed in a waveband of 7 to 12µm. The wavelength of 7 to 12µm is a waveband referred to as a growing beam and is effective for the growth of a plant or the like. In the culture of a stem cell capable of carrying out a self-growth and being differentiated into a plurality of advanced cells such as the cell of an animal, particularly, the human, for example, a cell having a growing ability such as a hepatic cell, a skin cell, an osteoblast or an immune cell, or a hematopoietic stem cell or an interstitial stem cell, if the growing beam is irradiated on the cell to be cultured, the growth and differentiation of the cell can be promoted more greatly as compared with the case in which the far infrared ray is not irradiated. As the cause for promoting the growth and differentiation of the cell, it can be supposed that a gene, a growth factor or a differentiation-inducing factor to influence the growth or the differentiation is activated, and contrarily, the action of the gene or a suppressor factor for suppressing the growth and differentiation is suppressed and a far infrared ray directly functions as the growth factor and the differentiation-inducing factor. If a cell which is undifferentiated and can be differentiated into the cells of various tissues, for example, an undifferentiated cell such as a stem cell derived from a bone marrow, a peripheral blood, a cord blood or a tissue or an embryonic stem cell is cultured in a state in which a far infrared ray is irradiated, it is a matter of course that the growth of an undifferentiated cell itself having a very high sensitivity can be promoted, and a differentiation from the undifferentiated cell to a desirable cell can also be promoted. Therefore, it is possible to easily culture a large amount of desirably differentiated cells from a small amount of undifferentiated cells. If a cell which has already been differentiated into each tissue and can be grown, for example, a hepatic cell or an osteoblast is cultured in a state in which the far infrared ray is irradiated, the growth can be activated by an action for a long period of time.

In other words, if the cell is cultured by using the incubator 1 according to the present embodiment, it is possible to enhance the growth and differentiation promotion efficiency of the cell more greatly as compared with a conventional incubator.

In the case in which the far infrared ray radiated from the surface of the planar heating member 11 has a high energy density in a predetermined wavelength, moreover, bacteria contained in the gas in the culture space 1h can be killed by the far infrared ray irradiated from the planar heating member 11. Consequently, the cell which is being cultured can be prevented from being contaminated by the bacteria or the like.

In addition, the far infrared ray is used. Even if the far infrared ray is directly irradiated on the cell which is being cultured, therefore, a bad influence is not given. Therefore, the bacteria sticking to the cell can also be caused to die. Consequently, the cell which is being cultured can be prevented from being contaminated more reliably. In other words, the temperature regulating means can also be caused to function as sterilizing means.

The planar heating member 11 is a far infrared ray radiating portion described in claims.

The sterilizing means may be provided separately from the planar heating member 11 of the temperature regulating means 10. In this case, the peak of the energy density of the far infrared ray can be regulated to be formed in the wavelength range described above. Therefore, it is possible to enhance the sterilizing effect of the sterilizing means.

Furthermore, the far infrared ray radiating portion does not need to be the planar heating member 11 as described above and is not particularly restricted if it can irradiate a far infrared ray having a predetermined wavelength on a cell.

Next, the planar heating member 11 will be described in detail.

Fig. 4 is a schematic view showing the simple body of the planar heating member 11 to be used in the incubator 1 according to the present embodiment, (A) being a schematic plan view and (B) being a sectional view taken along a line B - B in (A). In Fig. 4, the reference numeral 12 denotes a pair of upper and lower base materials formed of a material of polyethylene terephthalate (PET) like a film. The upper and lower base materials 12 and 12 have a watertightness and a property for transmitting a far infrared ray, and internal surfaces are stuck to each other in such a manner that liquid such as water does not permeate into their internal surfaces.

As shown in Fig. 4, the radiating portion 13 is sealed in a fluidtightness between the internal surfaces of the upper and lower base materials 12 and 12. The radiating portion 13 is formed by a conductive material such as a silver paste or a copper paste and includes an electrode portion 14 connected to power supply means 20 which will be described below. The electrode portion 14 is formed by printing the conductive material on the internal surfaces of the upper and lower materials 12 and 12, and includes a pair of electrodes 14a and 14b which are opposed to each other. The electrode portion 14 is connected to the power supply means 20 which will be described below.

A radiating member 15 is printed on the internal surfaces of the upper and lower base materials 12 and 12 between the electrodes 14a and 14b of the electrode portion 14 which are opposed to each other in order to come in contact with both of the electrodes 14a and 14b which are opposed to each other, respectively. The radiating member 15 includes a material for radiating a far infrared ray, for example, metal powder such as carbon black, carbon graphite, ceramics powder, alumina or zircon and a material having a positive temperature coefficient (P.T.C.) function of a semiconductor containing polyethylene glycol as a base material, a so-called self-temperature control function.

When a power is supplied from the power supply means 20, therefore, a voltage is applied between the opposed electrodes 14a and 14b of the electrode portion 14. Therefore, a current flows to the radiating member 15. Consequently, the PTC material and the radiating material generate heat by themselves so that temperatures are raised. Thus, it is possible to discharge a far infrared ray having a wavelength corresponding to the temperature from the radiating material.

In addition, the radiating member 15 is a material having a positive temperature coefficient. Even if a special control mechanism or sensor is not employed, therefore, the temperature of the PTC material or the radiating material can be maintained reliably in the vicinity of a predetermined temperature. Consequently, it is possible to maintain the wavelength of the far infrared ray radiated from the radiating member 15 and the distribution of the energy density in a predetermined state. Accordingly, it is possible to prevent a change in the state of the far infrared ray due to a variation in the temperature of the planar heating member 11, that is, to prevent a change in the temperature of a cell because the state of the far infrared ray to be radiated on the cell can be maintained to be constant. Thus, it is possible to culture the cell in a stable state.

The radiating portion 13 is formed by printing the conductive material or the radiating member 15 on the internal surfaces of the upper and lower base materials 12 and 12. Therefore, the planar heating member 11 can easily be manufactured inexpensively.

Moreover, the inner part of the incubator 1 is maintained in a state in which a humidity thereof is approximately 100%. For this reason, the watertightness of the planar heating member 11 becomes a very big problem. As shown in Fig. 4, however, the radiating portion 13 is sealed in a fluidtightness between the upper and lower base materials 12 and 12. Accordingly, it is possible to prevent water from permeating between the upper and lower base materials 12 and 12. Also in use for a long time on severe conditions in which a humidity is approximately 100% as in the inner part of the incubator 1, therefore, it is possible to prevent the generation of an electric leakage and a short circuit.

As shown in Fig. 4, by coating the surface of the base material 12 with a sheet formed by a material having a higher watertightness, for example, an olefin adhesive film, it is possible to further enhance the watertightness of the planar heating member 11.

In the case in which the planar heating member is used on a condition that the humidity is high as described above, how to prevent the permeation of the water into the connecting part of a portion for generating a heat and a power cord for supplying a power to the same portion is very important. In the planar heating member 11 employed in the present application, the power supply means 20 corresponding to a conventional power cord has the following structure. Consequently, the watertightness can be enhanced and the permeation of the water can be prevented reliably.

As shown in Fig. 4, the power supply means 20 includes a pair of upper and lower covering portions 21 and 21 and a conducting portion 22 provided between the upper and lower covering portions 21 and 21.

The upper and lower covering portions 21 and 21 are formed integrally with the upper and lower base materials 12 and 12, respectively. The upper and lower covering portions 21 and 21 have tips extended to have such a length as to be disposed on the outside of the culture space 1h when the planar heating member 11 is attached to the culture space 1h of the body 2 of the incubator 1. The upper and lower covering portions 21 and 21 have internal surfaces stuck to each other in such a manner that liquid such as water does not permeate into both of the internal surfaces in the same manner as the upper and lower base materials 12 and 12.

In the same manner as the electrode portion 14 of the radiating portion 13, moreover, the conducting portion 22 is formed by printing a conductive material on the internal surfaces of the upper and lower covering portions 21 and 21 and is sealed in a fluidtightness between the upper and lower covering portions 21 and 21. The conducting portion 22 has a base end portion connected to the electrode portion 14 of the radiating portion 13 and a tip portion provided in the tip portions of the upper and lower covering portions 21 and 21.

As described above, in the power supply means 20, the upper and lower covering portions 21 and 21 are formed integrally with the upper and lower base materials 12 and 12. Therefore, the connecting portion of the power supply means 20 and the radiating portion 13 can be reliably sealed in a fluidtightness from the outside. Even if the humidity in the culture space 1h approximates to 100%, consequently, it is possible to reliably prevent the water from permeating into the upper and lower base materials 12 and 12 from the connecting portion of both of them.

Moreover, the conducting portion 22 is also formed by the printing, and therefore, can be simultaneously formed together with the radiating portion 13. Consequently, it is possible to lessen the manufacturing man-hour of the planar heating member 11 and to manufacture the planar heating member 11 inexpensively. By using the same material as the material of the radiating portion 13 for the material of the conducting portion 22, particularly, it is possible to reduce a resistance in the connecting part of both of them and to easily form the conducting portion 22 because the same material is printed.

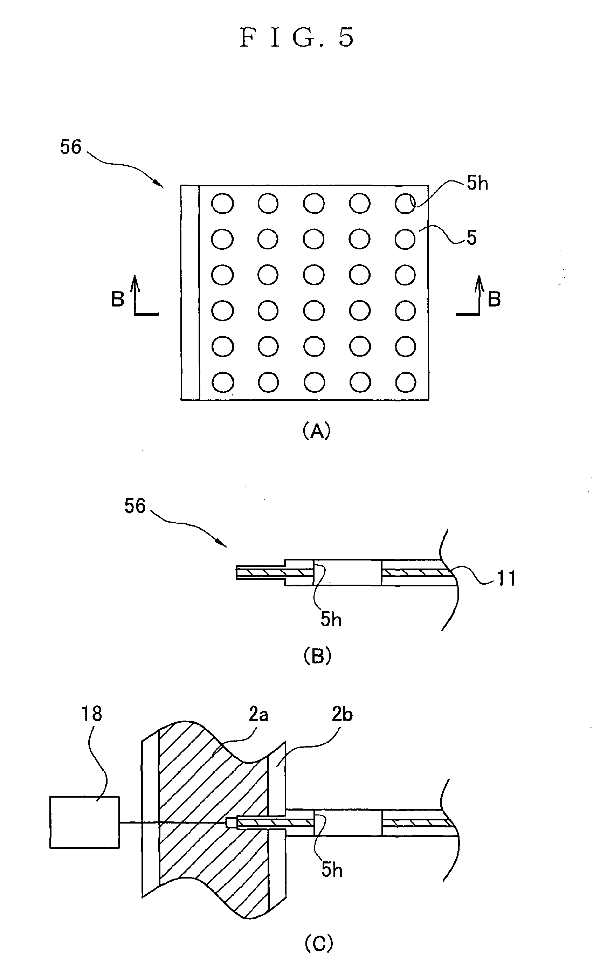

As shown in Figs. 1, 2 and 5, a sample holding member 5 on which a cell to be cultured is disposed may be provided in the culture space 1h of the body 2 in the incubator 1 and the planar heating member 11 may be provided in the sample holding member 5. In this case, if the sample holding member 5 is formed by a material for transferring a far infrared ray, the far infrared ray can be directly supplied to a cell without air. Consequently, it is possible to lessen the far infrared ray to be absorbed into steam in the culture space 1h. Thus, it is possible to maintain the temperature of the cell to be constant more reliably and to also increase an energy efficiency.

By regulating the wavelength of the far infrared ray to be radiated from the planar heating member 11 provided on each sample holding member 5, moreover, it is possible to adjust the wavelength of the far infrared ray to be irradiated on the cell for each sample holding member 5. Also in one culture space 1h, therefore, it is possible to culture the cell on different culture conditions.

In order to efficiently circulate the gas in the culture space 1h, each sample holding member 5 is provided with a plurality of through holes 5h penetrating vertically. In this case, in the planar heating member 11, it is preferable to provide through holes in corresponding places to the through holes 5h of the sample holding member 5.

If the sample holding member 5 is removably attached to the body 2, it is possible to adjust the area of the inner part of the culture space 1h or the like by regulating the number of the sample holding members 5 corresponding to a cell to be cultured or a container accommodating the cell.

In this case, a mechanism for supplying a power to the planar heating member 11 and a waterproof performance in that portion become problems. By using a terminal of a magnet type or a terminal of a pin type, however, it is possible to maintain a waterproofness.

Next, description will be given to a cell culturing method according to the present invention.

The cell culturing method according to the present invention serves to culture a cell to be cultured in a state in which the temperature of the cell itself is maintained in a predetermined temperature region, for example, 36.5 to 37.5□, and has a feature that a far infrared ray is irradiated on the cell during the culture.

In the cell culturing method according to the present invention, the temperature of the cell to be cultured can be raised by the far infrared ray to be irradiated in addition to a heat transfer from a gas in a space for culturing the cell and the temperature can be thus regulated. In other words, it is possible to regulate the temperature of the cell by an energy supplied by the far infrared ray in addition to a thermal energy supplied from the gas in the space for culturing the cell. Consequently, it is possible to suppress the influence of a change in the temperature of the gas on the temperature of the cell. Even if the temperature of the gas is changed, the temperature of the cell can be regulated to be almost constant. Thus, it is possible to culture the cell in a stable state.

By irradiating the far infrared ray on the cell to be cultured, it is possible to promote the growth and differentiation of the cell more greatly as compared with the case in which the far infrared ray is not irradiated. As the cause for promoting the growth and differentiation of the cell, it can be supposed that a gene, a growth factor or a differentiation-inducing factor to influence the growth or the differentiation is activated, and contrarily, the action of the gene or a suppressor factor for suppressing the growth and differentiation is suppressed and a far infrared ray directly functions as the growth factor and the differentiation-inducing factor. If a cell which is undifferentiated and can be differentiated into the cells of various tissues, for example, an undifferentiated cell such as a stem cell derived from a bone marrow, a peripheral blood, a cord blood or a tissue or an embryonic stem cell is cultured in a state in which a far infrared ray is irradiated, it is a matter of course that the growth of an undifferentiated cell itself having a very high sensitivity can be promoted, and a differentiation from the undifferentiated cell to a desirable cell can also be promoted. Therefore, it is possible to easily culture a large amount of desirably differentiated cells from a small amount of undifferentiated cells. If a cell which has already been differentiated into each tissue and can be grown, for example, a hepatic cell or an osteoblast is cultured in a state in which the far infrared ray is irradiated, the growth can be activated by an action for a long period of time.

In the case in which a hematopoietic stem cell is cultured, particularly, the growth and differentiation of the hematopoietic stem cell can be promoted at the same time by the far infrared ray. Consequently, a desirable differentiated cell such as a blood cell can be cultured easily in a large amount. For example, even if only a bone marrow cell in an amount of approximately several ml is cultured, the hematopoietic stem cell contained in the bone marrow cell grows and the growing hematopoietic stem cell is further differentiated into every blood cell. Therefore, it is possible to construct a blood system containing all blood cells.

When the hematopoietic stem cell and the interstitial stem cell are mixed and cultured, moreover, all differentiated blood cells can easily be cultured in a large amount. By extracting a desirable material component, that is, only a desirable blood cell from a culture solution, it is possible to easily manufacture a blood product containing a desirable blood component. The raw material of the blood product is a bond marrow cell in a very small amount. By extracting each of these components, therefore, it is possible to manufacture a whole blood preparation, an erythrocyte preparation, a blood plasma preparation, a blood platelet preparation, a blood coagulation factor preparation No. VIII, an albumin preparation, an immunoglobulin preparation and the like.

When the bone marrow cell is to be cultured, it is possible to obtain a blood cell or a blood component in a large amount more rapidly if a blood is mixed in addition to the culture solution. Therefore, it is possible to inexpensively manufacture each blood product in a larger amount.

### (Examples)

The state of the growth and differentiation of each cell was inspected in the case in which an incubator comprising a planar heating member for irradiating a far infrared ray according to the present invention (which will be hereinafter referred to as a far infrared CO₂ incubator) was used to culture a blood cell and an adhesive cell, and was compared with the state of the growth and differentiation of each cell in the case in which a general incubator, that is, an incubator having such a structure as to heat a gas in a culture space by means of the Nichrome wire and to regulate the temperature of the gas in a culture chamber was used to culture a blood cell and an adhesive cell.

Culture conditions were set to be identical in all incubators, and the gas in the culture space was set to have a temperature of 37°C and a humidity of 100% and the volume rate of a gas component was set to have 95% of air and 5% of carbon dioxide. Moreover, MEM (SIGMA, Irvine KA12, UK) containing 1% of antibiotic (GIBCO, N.Y., USA) and 50% of blood serum (FETAL BOVINE SERUM (SGMA, Irvine KA12, UK) ) in a volume rate was used for a culture solution.

For the culture solution, furthermore, the amount of the blood to be added having a mass rate to the whole culture solution of 0 to 30% was changed and the influence of the amount of the blood to be added to the culture solution on a culture was also inspected.

### (Example 1)

In the case in which a hematopoietic stem cell was cultured, the increase and decrease of the number of red blood cells and hemoglobins contained in the culture solution was inspected.
(1) The bone marrow of a rabbit (Japan White, 6 to 7 age in week, KITAYAMA LABES CO., LTD., Ina JAPAN) was suspended in a culture solution in an amount of 20ml (DULBECCO'S MODIFIED NUTRIENT MIXTURE F-12 HAM containing (SIGMA, Irvine KA12, UK), 1% Antibiotic - Antimycotic (GIBCO, N.Y., USA)).
(2) The suspension was divided equally into twelve 50 ml tubes (Falcon, NJ, USA).
(3) The bone marrow cell was sedimented by means of a centrifugal separator (SAKUMA R300S-11, Tokyo) (1000 rpm, 5 min) and only a supernatant was absorbed.
(4) The blood and the culture solution were mixed to prepare a solution in 60ml and the bone marrow cell sedimented by a centrifugal operation was added in order to obtain a blood concentration as will be described below.

**[Table 1]**

| |
|---|
| Blood concentration |
| Blood |
| Culture solution |

(5) Two 24-hole plates (Nunk, Denmark) (14 in total) were prepared and 1 ml was scattered in each of three places, and a sample was used for seven days in total, that is, 0th, first, third, fifth, seventh, tenth and 14th days. In order to eliminate a supposed error, a measurement was carried out in three places a day and an average was taken.
(6) One plate was put in an infrared CO₂ incubator (obtained by improving a product of NAPCO, IL, USA) and one plate was put in the Nichrome wire CO₂ incubator (NAPCO, IL, USA), and they were left. Each sample was mixed well by means of a pipette after 0, 1, 3, 5, 7, 10 and 14 days and was moved to an eppendorf tube (eppendorf, UK).
(7) The number of red blood cells in each sample was measured three times by using a blood cell number automatic measuring apparatus (Sysmex K-4500, Kobe).

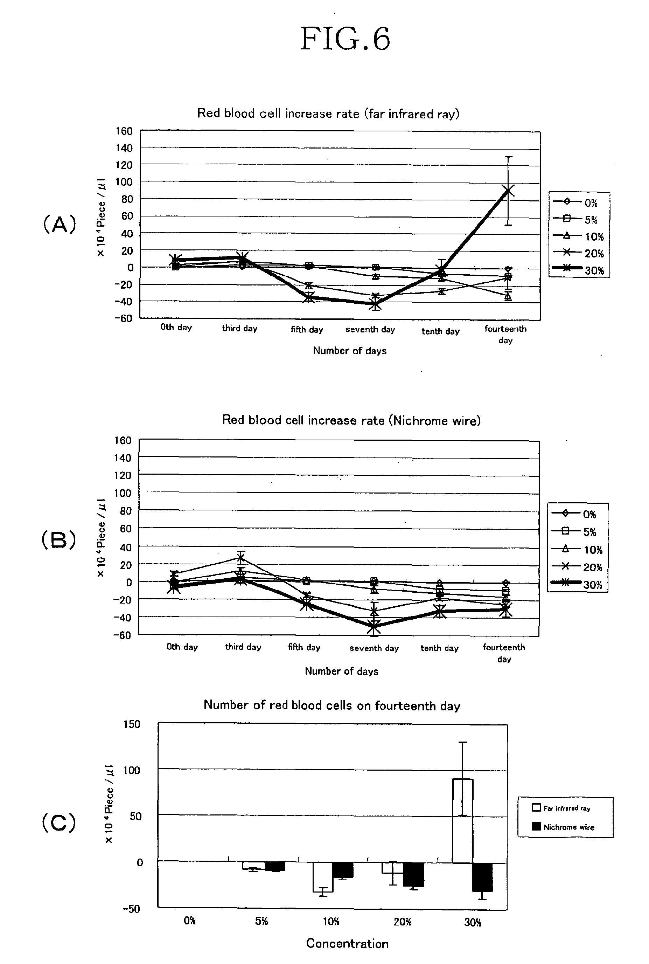

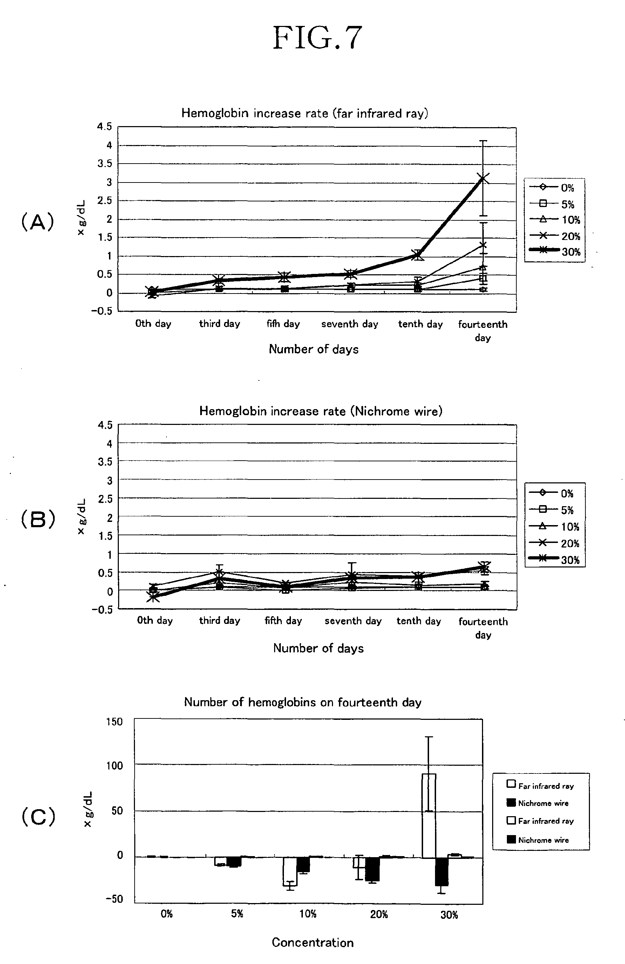

As shown in Figs. 6 and 7, when a far infrared ray was irradiated, a red blood cell was produced more actively in a blood concentration of 20% and 30% as compared with that in a reference group. With a blood concentration of 30% on the 14th day for a culture, -9.88 × 10⁴ pieces /µl was reached for the reference group, while 91.12 × 10⁴ pieces /µl was reached.

Moreover, the hemoglobin was increased depending on the blood concentration with the passage of time in the reference group and the far infrared ray irradiation group, and was increased to be approximately 4.8 times as great as that in the reference group with the blood concentration of 30% on the 14th day for the culture.

### (Example 2)

In the case in which a hematopoietic stem cell was cultured, the increase and decrease of the number of white blood cells and hematopoietic stem cells contained in the culture solution was inspected.
(1) The bone marrow of a rabbit (Japan White, 6 to 7 age in week, KITAYAMA LABES CO., LTD., Ina JAPAN) was suspended in a culture solution in an amount of 20ml (DULBECCO'S MODIFIED NUTRIENT MIXTURE F-12 HAM containing (SIGMA, Irvine KA12, UK), 1% Antibiotic - Antimycotic (GIBCO, N.Y., USA)).
(2) The suspension was divided equally into twelve 50 ml tubes (Falcon, NJ, USA).
(3) The bone marrow cell was sedimented by means of a centrifugal separator (SAKUMA R300S-11, Tokyo) (1000 rpm, 5 min) and only a supernatant was absorbed.
(4) The blood and the culture solution were mixed to prepare a solution in 60ml and the bone marrow cell sedimented by a centrifugal operation was added in order to obtain a blood concentration as will be described below.

**[Table 2]**

| |
|---|
| Blood concentration |
| Blood |
| Culture solution |

(5) Two 24-hole plates (Nunk, Denmark) (14 in total) were prepared and 1 ml was scattered in each of three places, and a sample was used for seven days in total, that is, 0th, first, third, fifth, seventh, tenth and 14th days. (In order to eliminate an error, a measurement was carried out in three places a day and an average was taken.)
(6) One plate was put in an infrared CO₂ incubator (obtained by improving a product of NAPCO, IL, USA) and one plate was put in the Nichrome wire CO₂ incubator (NAPCO, IL, USA), and they were left. Each sample was mixed well by means of a pipette after 0, 1, 3, 5, 7, 10 and 14 days and was moved to an eppendorf tube (eppendorf, UK).
(7) The numbers of the white blood cells and the bone marrow cells in each sample were measured three times by using a blood cell number automatic measuring apparatus (Sysmex K-4500, Kobe) .

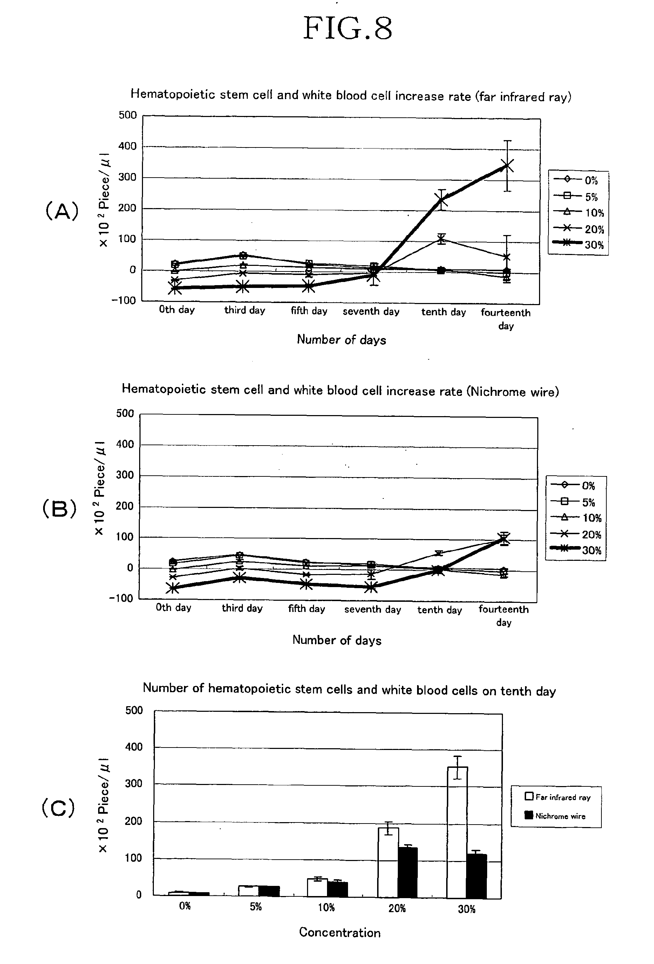

As shown in Fig. 8, the white blood cell and the hematopoietic stem cell were increased depending on the blood concentration with the passage of time in the reference group and the far infrared ray irradiation group in a blood concentration of 20% or less, and were increased in the far infrared ray irradiation group to be approximately 3.0 times as great as that in the reference group with a blood concentration of 30% on the 10th day for the culture.

### (Example 3)

In the case in which a hematopoietic stem cell was cultured, the increase and decrease of blood platelets contained in the culture solution was inspected.
(1) The bone marrow of a rabbit (Japan White, 6 to 7 age in week, KITAYAMA LABES CO., LTD., Ina JAPAN) was suspended in a culture solution in an amount of 20ml (DULBECCO'S MODIFIED NUTRIENT MIXTURE F-12 HAM containing (SIGMA, Irvine KA12, UK), 1% Antibiotic - Antimycotic (GIBCO, N.Y., USA)).
(2) The suspension was divided equally into twelve 50 ml tubes (Falcon, NJ, USA).
(3) The bone marrow cell was sedimented by means of a centrifugal separator (SAKUMA R300S-11, Tokyo) (1000 rpm, 5 min) and only a supernatant was absorbed.
(4) The blood and the culture solution were mixed to prepare a solution in 60ml and the bone marrow cell sedimented by a centrifugal operation was added in order to obtain a blood concentration as will be described below.

**[Table 3]**

| |
|---|
| Blood concentration |
| Blood |
| Culture solution |

A blood serum was not contained in the culture solution which was used.
(5) Two 24-hole plates (Nunk, Denmark) (14 in total) were prepared and 1 ml was scattered in each of three places, and a sample was used for seven days in total, that is, 0th, first, third, fifth, seventh, tenth and 14th days. (D In order to eliminate an error, a measurement was carried out in three places a day and an average was taken.)
(6) One plate was put in an infrared CO₂ incubator (obtained by improving a product of NAPCO, IL, USA) and one plate was put in the Nichrome wire CO₂ incubator (NAPCO, IL, USA), and they were left. Each sample was mixed well by means of a pipette after 0, 1, 3, 5, 7, 10 and 14 days and was moved to an eppendorf tube (eppendorf, UK).
(7) The number of the blood platelets in each sample was measured three times by using a blood cell number automatic measuring apparatus (Sysmex K-4500, Kobe).

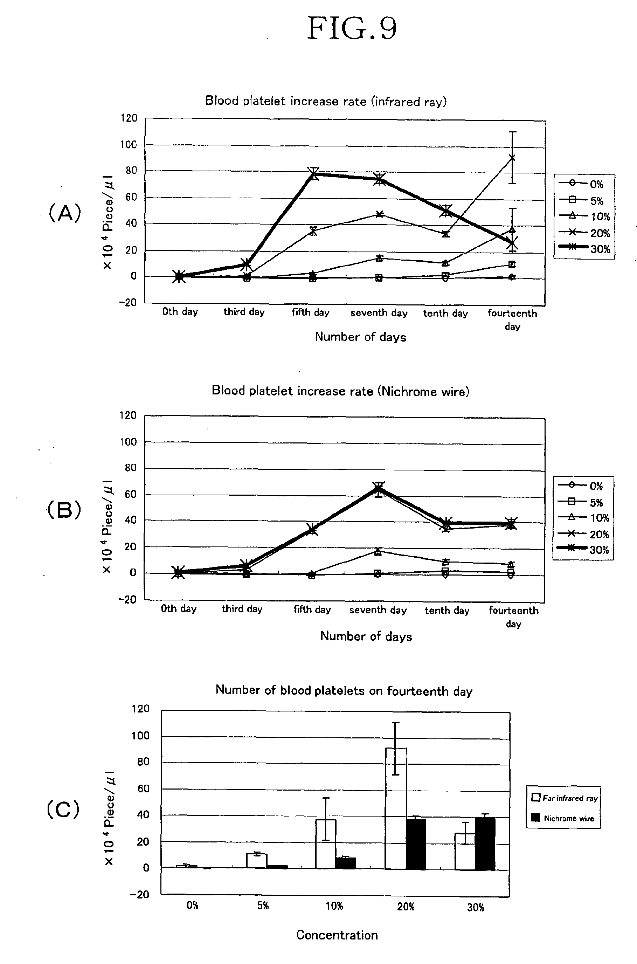

As shown in Fig. 9, it was apparent that the blood platelet is increased depending on the blood concentration in the reference group and the far infrared ray irradiation group in a blood concentration of 20% or less and is decreased with a peak in 5 to 7 days, and furthermore, is increased after 10 days. The amount of production of the blood platelet in the far infrared ray irradiation group was increased to be approximately 2.4 times as great as that in the reference group with a blood concentration of 20% on the 14th day for the culture.

### (Example 4)

In the case in which a hematopoietic stem cell and an adhesive cell were cultured, the increase and decrease of the number of adhesive cells was inspected.
(1) The bone marrow of a rabbit (Japan White, 6 to 7 age in week, KITAYAMA LABES CO., LTD., Ina JAPAN) was suspended in a culture solution in an amount of 20ml (DULBECCO'S MODIFIED NUTRIENT MIXTURE F-12 HAM containing (SIGMA, Irvine KA12, UK), 1% Antibiotic - Antimycotic (GIBCO, N.Y., USA)).
(2) The suspension was divided equally into twelve 50 ml tubes (Falcon, NJ, USA).
(3) The bone marrow cell was sedimented by means of a centrifugal separator (SAKUMA R300S-11, Tokyo) (1000 rpm, 5 min) and only a supernatant was absorbed.
(4) The blood and the culture solution were mixed to prepare a solution in 60ml and the bone marrow cell sedimented by a centrifugal operation was added in order to obtain a blood concentration as will be described below.

**[Table 4]**

| |
|---|
| Blood concentration |
| Blood |
| Culture solution |

(5) Two 24-hole plates (Nunk, Denmark) (14 in total) were prepared and 1 ml was scattered in each of three places, and a sample was used for seven days in total, that is, 0th, first, third, fifth, seventh, tenth and 14th days. (□ In order to eliminate an error, a measurement was carried out in three places a day and an average was taken.)
(6) One plate was put in an infrared CO₂ incubator (obtained by improving a product of NAPCO, IL, USA) and one plate was put in the Nichrome wire CO₂ incubator (NAPCO, IL, USA), and they were left. A suspended cell solution was thrown away after 0, 1, 3, 5, 7, 10 and 14 days.
(7) 400µl of Hank' s Balanced Salt Solution (GIBCO, N.Y., USA) was added to the empty plate and the plate was washed, and the same solution was absorbed.
(8) 300µl of Trypsin-EDTA (GIBCO, N.Y. , USA) was put and left for a while, and a cell was peeled by a pipette operation and a blood serum was added in an amount of 100µl.
(9) The cell was moved to an eppendorf tube (eppendorf, UK) and was measured by a hemacytometer (Sunlead Glass, Tokyo) and a blood cell number automatic measuring apparatus (Sysmex K-4500, Kobe), respectively.

The number of the peeled cells was counted in the following procedure by using the hemacytometer.

First of all, a cover glass was put on a calculating board and a culture cell was injected from a clearance, and the number of the cells was counted by means of a microscope. In consideration of an injection error, the counting was carried out five times. If the number of the cells in four sides is represented as A, a cell concentration to be obtained is A X 10⁴ pieces / ml.

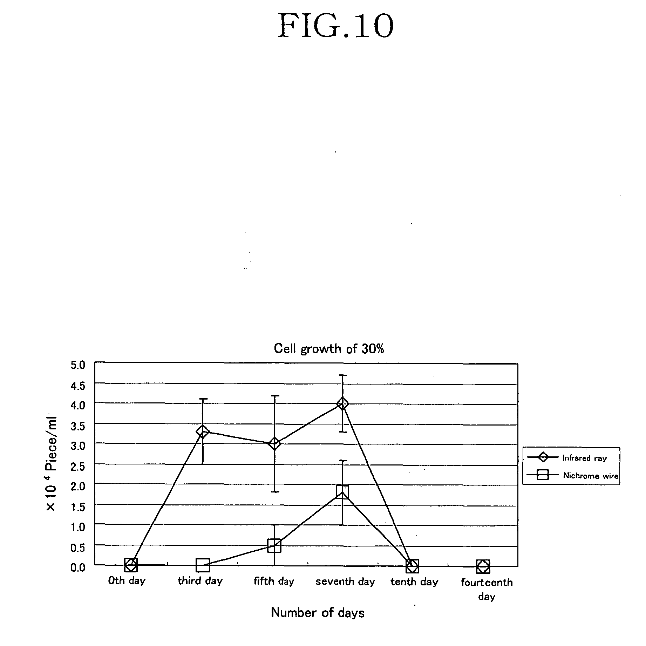

As shown in Fig. 10, a significant difference in the adhesive cell was not observed in a blood concentration of 20% or less. However, the adhesive cell was increased more significantly in all samples with a blood concentration of 30% in 3 to 10 days for the culture in the far infrared ray irradiation group than that in the reference group. After 10 days for the culture, the culture solution could not be exchanged. For this reason, the cell died.

### (Example 5)

In the case in which only an adhesive cell was cultured, the increase and decrease of the number of the adhesive cells was inspected.
(1) 1000000 cells of three types including ST-2 (the Physical and Chemical Research Institute Gene Bank, Tsukuba), MC3T3-E1 (the Physical and Chemical Research Institute Gene Bank, Tsukuba), and C3H10T1/2 (Research Resource Bank, Osaka) which are mouse osteoblasts were suspended well by the addition of a culture solution in an amount of 18ml {ST-2 : RPMI1640 (GIBCO, N.Y., USA) 10% FETAL BOVINE SERUMD (SIGMA, Irvine KA12, UK), 1% Antibiotic-Antimycotic (GIBCO, N.Y., USA), MC3T3-E1 : DULBECCO'S MODIFIED EAGLE'S MEDIUM NUTRIENT MIXTURE F-12 HAM (SIGMA, Irvine KA12, UK), 10% FETAL BOVINE SERUMD (SIGMA, Irvine KA12, UK), 1% Antibiotic-Antimycotic (GIBCO, N.Y., USA), C3H10T1/2 : BASAL MEDIUM OF EAGLE WITHEARLE'S SALTS (GIBCO, N.Y., USA), 10%FETAL BOVINE SERUMD (SIGMA, Irvine KA12, UK), 1% Antibiotic-Antimycotic (GIBCO, N.Y., USA) }.
(2) 400µl of the culture solution was scattered to one hole of a 24-hole plate (Nunk, Denmark).
(3) 600µl of the culture solution was added to one hole after 30 minutes. One hole of the 24-hole plate has a capacity of approximately 1 ml.
(4) Culture was started in a Nichrome wire CO₂ incubator (NAPCO, IL, USA) and a far infrared CO₂ incubator (obtained by improving a product of NAPCO, IL, USA).
(5) The number of cells was measured three times a day (once for one hole) on 0th, first, third, fifth, seventh, tenth and 14th days.

The number of the cultured cells was measured in the following procedure.
(1) The culture solution was absorbed and 400µl of Hank's Balanced Salt Solution (GIBCO, N.Y., USA) was put and absorbed again.
(2) 300µl of Trypsin-EDTA (GIBCO, N.Y., USA) was put and left for a while (approximately 5 minutes), and the adhesive cell was peeled by a pipette operation and a blood serum was added in an amount of 100µl, and the pipetting was carried out well again.
(3) The solution was moved to an eppendorf tube (eppendorf, UK) and was measured three to four times by using a hemacytometer (Sunlead Glass, Tokyo) and three to four times by using a blood cell automatic measuring apparatus (Sysmex K-4500, Kobe). A measurement was carried out in three places a day and an average was taken in order to eliminate an error.

As shown in Fig. 10, a significant difference between the far infrared ray irradiation group and the reference group was not observed in 14 days for the culture in all of the cells of the three types including ST-2, MC3T3-E1 and C3H10T1/2 to be the mouse osteoblasts.

### Industrial Applicability

The incubator and the cell culturing method according to the present invention are suitable for culturing a blood cell such as a red blood cell, a white blood cell or a blood platelet or other adhesive cells.

### Numerical list of the drawings

FIG. 1
   - (1): incubator
   - (2): body
   - (3): door
   - (5): sample holding member
FIG. 2
   - (1): incubator
   - (2): body
   - (3): door
   - (1h): culture space
   - (11): planar heating member
   - (5): sample holding member
FIG. 3
   - (2): body
   - (3): door
   - (11): planar heating member
   - (10): temperature regulating means
FIG. 4
   - (11): planar heating member
   - (12): base material
   - (20): power supply means
   - (15): radiating member
   - (13): radiating portion
FIG. 6
   - (A): Red blood cell increase rate (far infrared ray)
   Piece
   0th day
   third day
   fifth day
   seventh day
   tenth day
   fourteenth day
   Number of days
   - (B): Red blood cell increase rate (Nichrome wire)
   Piece
   0th day
   third day
   fifth day
   seventh day
   tenth day
   fourteenth day
   Number of days
   - (C): Number of red blood cells on fourteenth day
   Piece
   Concentration
   Far infrared ray
   Nichrome wire
FIG. 7
   - (A): Hemoglobin increase rate (far infrared ray)
   0th day
   third day
   fifth day
   seventh day
   tenth day
   fourteenth day
   Number of days
   - (B): Hemoglobin increase rate (Nichrome wire)
   0th day
   third day
   fifth day
   seventh day
   tenth day
   fourteenth day
   Number of days
   - (C): Number of hemoglobins on fourteenth day
   Concentration
   Far infrared ray
   Nichrome wire
   Far infrared ray
   Nichrome wire
FIG. 8
   - (A): Hematopoietic stem cell and white blood cell increase rate (far infrared ray)
   Piece
   0th day
   third day
   fifth day
   seventh day
   tenth day
   fourteenth day
   Number of days
   - (B): Hematopoietic stem cell and white blood cell increase rate (Nichrome wire)
   Piece
   0th day
   third day
   fifth day
   seventh day
   tenth day
   fourteenth day
   Number of days
   - (C): Number of hematopoietic stem cells and white blood cells on tenth day
   Piece
   Concentration
   Far infrared ray
   Nichrome wire
FIG. 9
   - (A): Blood platelet increase rate (infrared ray)
   Piece
   0th day
   third day
   fifth day
   seventh day
   tenth day
   fourteenth day
   Number of days
   - (B): Blood platelet increase rate (Nichrome wire)
   Piece
   0th day
   third day
   fifth day
   seventh day
   tenth day
   fourteenth day
   Number of days
   - (C): Number of blood platelets on fourteenth day
   Piece
   Concentration
   Far infrared ray
   Nichrome wire
FIG. 10
   Cell growth of 30%
   Piece
   0th day
   third day
   fifth day
   seventh day
   tenth day
   fourteenth day
   Number of days
   Infrared ray
   Nichrome wire
FIG. 11
   - (A): Piece
   0th day
   third day
   seventh day
   ninth day
   fourteenth day
   Number of days
   Far infrared ray
   Nichrome wire
   - (B): Piece
   0th day
   third day
   seventh day
   ninth day
   fourteenth day
   Number of days
   Far infrared ray
   Nichrome wire
   - (C): Piece
   0th day
   third day
   seventh day
   ninth day
   fourteenth day
   Number of days
   Far infrared ray
   Nichrome wire

## Claims

1. An incubator comprising environment regulating means having a culture space which can be sealed and serving to maintain an internal environment in the culture space into a predetermined state, the environment regulating means of the apparatus including temperature regulating means for regulating a temperature in the culture space, the temperature regulating means having a far infrared ray radiating portion for radiating a far infrared ray.

2. The incubator according to claim 1, comprising environment regulating means having a culture space which can be sealed and serving to maintain an internal environment in the culture space into a predetermined state, the environment regulating means of the apparatus including sterilizing means for carrying out a sterilization in the culture space, the sterilizing means having a far infrared ray radiating portion for radiating a far infrared ray.

3. The incubator according to claim 1 or 2, wherein the far infrared ray radiating portion has a peak of an energy density of a far infrared ray to be radiated in a waveband of 7 to 12µm.

4. The incubator according to claim 1, 2 or 3, wherein the far infrared ray radiating portion is a planar heating member.

5. The incubator according to claim 4, wherein the planar heating member is constituted by a pair of upper and lower base materials formed by a material having a waterproofness, a radiating portion sealed in a fluidtightness between the upper and lower base materials and serving to radiate a far infrared ray when it is conducted, and power supply means for supplying a power to the radiating portion, and the radiating portion is formed on internal surfaces of the upper and lower base materials by printing a radiating member.

6. The incubator according to claim 5, wherein the radiating member is a material having a positive temperature coefficient.

7. The incubator according to claim 5 or 6, wherein the power supply means is constituted by a covering portion formed integrally with the upper and lower base materials and having a tip extended to an outside of the culture space, and a conducting portion provided from the tips of the pair of covering portions to the radiating portion, and the conducting portion is formed on the internal surfaces of the upper and lower covering portions by printing a material having a conductivity, and a portion between a tip portion and the radiating portion is sealed in a fluidtightness between the pair of covering portions.

8. The incubator according to claim 1, 2, 3, 4, 5, 6 or 7, wherein the culture space of the apparatus is provided with a sample holding member on which a culturing object is to be disposed, and the sample holding member includes the far infrared ray radiating portion.

9. The incubator according to claim 8, wherein the sample holding member is removably attached to the apparatus.

10. A cell culturing method for culturing a cell to be cultured in a state in which a temperature thereof is held in a predetermined temperature region, wherein a far infrared ray is irradiated on the cell.

11. The cell culturing method according to claim 10, wherein the cell is an animal cell, a viral infection cell or a gene recombination cell.

12. The cell culturing method according to claim 10, wherein the cell is an undifferentiated cell or a juvenile cell and has a pluripotency or a differentiation potency.

13. The cell culturing method according to claim 10, wherein the undifferentiated cell is a stem cell derived from a bone marrow, a peripheral blood, a cord blood or a tissue, a cell in a differentiating process or having a differentiation potency, or an embryonic stem cell.

14. A blood product comprising material and immaterial components of a blood which is cultured, wherein the material and immaterial components of the blood irradiate a far infrared ray on a hematopoietic stem cell, and the hematopoietic stem cell is differentiated and produced.
